(19) Europäisches Patentamt
European Patent Office
Office européen des brevets

(11) Veröffentlichungsnummer : **0 470 375 B1**

(12) **EUROPÄISCHE PATENTSCHRIFT**

(45) Veröffentlichungstag der Patentschrift : **23.03.94 Patentblatt 94/12**

(51) Int. Cl.$^5$ : **C07C 213/00,** C07C 209/36

(21) Anmeldenummer : **91111274.6**

(22) Anmeldetag : **06.07.91**

(54) **Verfahren zur Herstellung von in p-Stellung durch C1-C4-alkoxysubstituierten aromatischen Aminen.**

(30) Priorität : **20.07.90 DE 4023056**

(43) Veröffentlichungstag der Anmeldung : **12.02.92 Patentblatt 92/07**

(45) Bekanntmachung des Hinweises auf die Patenterteilung : **23.03.94 Patentblatt 94/12**

(84) Benannte Vertragsstaaten : **BE CH DE FR GB IT LI NL SE**

(56) Entgegenhaltungen :
**DE-A- 2 617 808**
**FR-A- 2 555 575**
**US-A- 3 953 510**

(56) Entgegenhaltungen :
**CHEMICAL ABSTRACTS, vol. 88, no. 23, 5. Juni 1978, Columbus, Ohio, US; abstract no. 169698M, V.S.TSENYUGA ET.AL.: 'Catalytic reduction of nitrobenzene in aliphatic alcohol-sulfuric acid media' Seite 551 ;Spalte 2 ;**
**Houben-Weyl VI/1C 91-92 (1976)**
**P.N. RYLANDER, Catalytic Hydrogenation over Platinum Metals, Academic Press 1967, p. 42/43**

(73) Patentinhaber : **BAYER AG**
**D-51368 Leverkusen (DE)**

(72) Erfinder : **Landscheidt, Heinz, Dr.**
**Liliencronstrasse 6**
**W-4100 Duisberg 1 (DE)**
Erfinder : **Klausener, Alexander, Dr.**
**Weissdornweg 37**
**W-5190 Stolberg (DE)**
Erfinder : **Blank, Heinz-Ulrich, Dr.**
**Am Geusfelde 35**
**W-5068 Odenthal (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft die Herstellung von aromatischen Aminen, die in p-Stellung durch $C_1$-$C_4$-Alkoxy substituiert sind, durch katalytische Hydrierung der zugrunde liegenden aromatischen Nitroverbindungen in einem Reaktionsmedium aus Schwefelsäure und einem $C_1$-$C_4$-Alkohol im Sinne der sogenannten Bamberger-Umlagerung.

Bei der Bamberger-Umlagerung wird beispielsweise aus Phenylhydroxylamin unter der Einwirkung von Mineralsäuren und Eintritt von Methanol als nukleophilem Agens p-Methoxy-anilin gebildet. Wie aus dem folgenden Formelschema hervorgeht, kann als nukleophiles Agens auch Wasser in die p-Stellung unter Bildung von p-Amino-phenol eintreten; weiterhin kann der Eintritt des nukleophilen Agens auch in die o-Position zur ursprünglichen Hydroxylamingruppe erfolgen. Seit langem hat man diese Bamberger-Umlagerung mit der intermediären Bildung des Hydroxylamins aus der zugrunde liegenden Nitroverbindung durch katalytische Hydrierung oder elektrochemische Reduktion verknüpft. Die am meisten beschriebene und für technische Anwendungen allein in Frage kommende Mineralsäure ist hierbei die Schwefelsäure. Bei einer solchen Zusammenfassung der Hydrierung der Nitrogruppe mit der Bamberger-Umlagerung kann als weitere Nebenreaktion die Bildung der nicht weiter substituierten Aminoverbindung aus der zugrunde liegenden Nitroverbindung eintreten. Diese Vorgänge lassen sich zusammenfassend wie folgt formelmäßig am Beispiel der Reduktion und Umsetzung von Nitrobenzol darstellt:

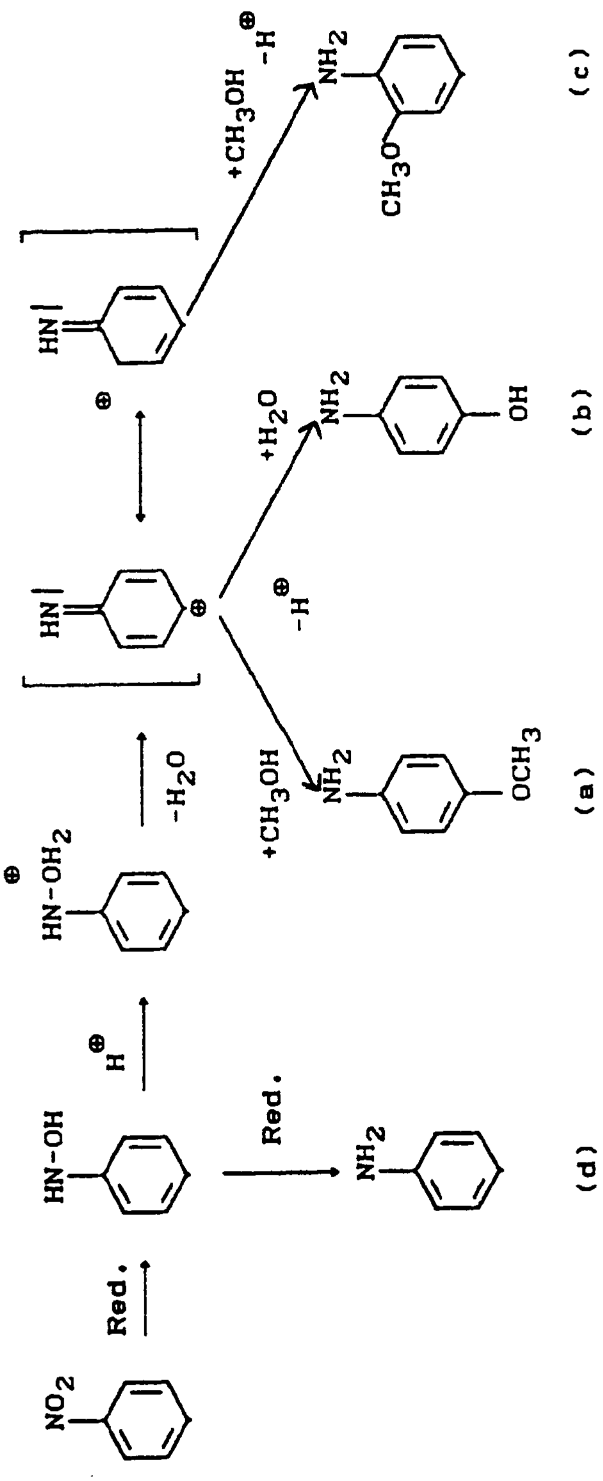


Das in überwiegender Menge in schwefelsaurer/methanolischer Umgebung entstehende p-Methoxy-anilin (a) ist das im allgemeinen angestrebte Produkt. Daneben treten als Nebenprodukte p-Amino-phenol (b), o-Methoxy-anilin (c) und Anilin (d) auf. Das letztere (d) entsteht hierbei offensichtlich durch Konkurrenz zur Bamberger-Verbindung durch Weiterreduktion des Phenylhydroxylamins.

Als weitere unerwünschte Nebenreaktion tritt Etherbildung aus dem eingesetzten Alkohol auf, dies insbesondere, da die mit der Reduktion der Nitroverbindung verbundene Bamberger-Umlagerung eine mehrstündige Reaktion darstellt und dies auch um so mehr, da zur Vermeidung einer noch längeren Reaktion im allgemeinen bei erhöhter Temperatur gearbeitet wird.

Es ist bekannt, daß Nitroaromaten, wie beispielsweise Nitrobenzol, 2- und 3-Nitro-toluol, 2,3-Dinitro-toluol oder 6-Chlor-2-nitrotoluol, in Methanol und in Gegenwart von Schwefelsäure oder Methylschwefelsäure elektrochemisch zu 4-Alkoxy-aminoaromaten reduziert werden können (vgl. DE-OS 2617808, JP 55154590, Kagaku Kogyo 56 293-296 (1982)). Derartige Verfahren verursachen hohe Energiekosten und einen beträchtlichen apparativen Aufwand.

Ferner ist bekannt, daß Nitroaromaten, wie beispielsweise 2-Chlornitrobenzol, 2-Nitro-toluol oder 2,6-Dimethyl-nitrobenzol, in alkoholischer Lösung unter Zusatz von Wasser oder Carbonsäuren bei einem Wasserstoffdruck druck zwischen 0,001 und 0,1 MPa (0,01 und 1 bar) zu 4-Alkoxy-aminoaromaten umgesetzt werden können (vgl. DE-OS 3443385 und JP 61109759). Wie aus der Literatur bekannt ist, muß bei Gegenwart von Wasser mit der unerwünschten Bildung von p-Hydroxy-aminoaromaten im Sinne einer konkurrierenden klassischen Bamberger-Reaktion in wäßrigen Reaktionsmedien gerechnet werden (vgl, HOUBEN-WEYL VI/1c, 91-92). Der Zusatz von Carbonsäuren ist unter wirtschaftlichen Gesichtspunkten unvorteilhaft.

Es ist weiterhin bekannt, daß verschiedene Nitroaromaten, wie beispielsweise Nitrobenzol, in alkoholischer Lösung und in Gegenwart von Schwefelsäure unter Zusatz von Katalysatorgiften wie beispielsweise Dimethylsulfoxid oder unter Verwendung modifizierter und desaktivierter Katalysatoren, wie Platinoxid oder Platinsulfid, bei Drücken von bis zu 0,6 MPa (6 bar) Wasserstoff zu 4-Alkoxyaminoaromaten umgesetzt werden können (vgl. SU 523081, SU 520347, SU 514811 und SU 578302, Zh. Org. Khim. 1978, 14(2), 337-9, zitiert nach C.A. 88 (1978), 169 698 m, sowie Nippon Kagaku Kaishi 1237 (1982), 245 (1980) und 1532 (1979)). Derartige modifizierte Katalysatoren sind teuer und zumeist nicht oder nur sehr begrenzt wiederverwendungsfähig, was einen erheblichen wirtschaftlichen Nachteil darstellt. Der Zusatz von Moderatoren bzw. Inhibitoren wie Dimethylsulfoxid erschwert darüber hinaus die Aufarbeitung und Reinigung der rohen Reaktionsprodukte und führt aufgrund der Desaktivierung der Edelmetallkatalysatoren zur Verlängerung der Reaktionszeiten und somit zu ungünstigen Raum-Zeit-Ausbeuten.

Nach Literaturangaben muß bei Durchführung von Bamberger-Reaktionen in alkoholischen Reaktionsmedien zum Zwecke der Darstellung von p-Alkoxy-aminoaromaten im Falle der Anwendung höherer Wasserstoffdrücke als etwa 0,02 bis 0,2 MPa (0,2-2 bar) Wasserstoffdruck damit gerechnet werden, daß überwiegend die durch einfache Reduktion der Nitrogruppen entstehenden Aminoaromaten oder gar kernhydrierte Verbindungen erhalten werden. Um dies zu verhindern, vermeidet man im allgemeinen höhere Drücke und nimmt teilweise erheblich verlängerte Reaktionszeiten in Kauf (vgl. DE-OS 3 443 385, JP 53084925, 57072945 und 57002247).

Es wurde nun gefunden, daß es überraschenderweise deutliche Vorteile ergibt, diese Reaktion unter erhöhtem Druck, insbesondere unter erhöhtem Wasserstoff-Partialdruck und vorteilhafterweise auch bei erhöhter Temperatur und auch in Gegenwart nicht desaktivierter Katalysatoren durchzuführen. Dies ist überraschend, weil unter solchen Bedingungen nach fachmännischem Wissen bereits das Einsetzen einer Durchhydrierung des aromatischen Kerns zum cycloaliphatischen Ring überhand nehmen sollte. In weiterhin überraschender Weise wird beim erfindungsgemäßen Verfahren außerdem die befürchtete Etherbildung trotz der bevorzugt erhöhten Temperatur stark unterdrückt. Der wesentliche Vorteil des erfindungsgemäßen Verfahrens ist die stark verkürzte Reaktionszeit, die nur einen Bruchteil der von Verfahren des Standes der Technik beträgt, womit sich die Apparateausnutzung (Raum-Zeit-Ausbeute) beträchtlich erhöht.

Es wurde ein Verfahren zur Herstellung von in p-Stellung durch $C_1$-$C_4$-Alkoxy substituierten aromatischen Aminen der Formel (I)

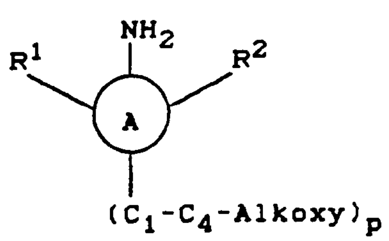

(I) ,

in der

| | |
|---|---|
| A | für den Benzol- oder den Naphthalinkern steht, |
| p | die p-Stellung zur Aminogruppe anzeigt und |
| $R^1$ und $R^2$ | unabhängig voneinander Wasserstoff, $C_1$-$C_4$-Alkyl, $C_1$-$C_4$-Alkoxy oder Halogen bedeuten, |

durch katalytische Hydrierung einer aromatischen Nitroverbindung in einem Reaktionsmedium aus Schwefelsäure und einem $C_1$-$C_4$-Alkohol bei erhöhter Temperatur gefunden, das dadurch gekennzeichnet ist, daß als Nitroverbindung eine der Formel (II)

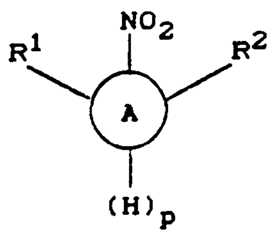

(II) ,

in der A, p, $R^1$ und $R^2$ die obige Bedeutung haben, unter erhöhtem Druck, von dem der Wasserstoff-Partialdruck 0,3 bis 10 MPa (3 bis 100 bar) beträgt, in Gegenwart nicht desaktivierter Katalysatoren aus der Platinmetall-Gruppe umgesetzt wird, wobei 5 bis 100 Mol des Alkanols und 0,8 bis 2,5 Mol Schwefelsäure, jeweils pro Mol der aromatischen Nitroverbindung, eingesetzt werden.

$C_1$-$C_4$-Alkyl ist beispielsweise Methyl, Ethyl, Propyl, Isopropyl, Butyl, Isobutyl, bevorzugt Methyl oder Ethyl, besonders bevorzugt Methyl.

$C_1$-$C_4$-Alkoxy ist beispielsweise Methoxy, Ethoxy, Propoxy, Isopropoxy, Butoxy, Isobutoxy, bevorzugt Methoxy oder Ethoxy, besonders bevorzugt Methoxy.

Halogen ist beispielsweise Fluor, Chlor oder Brom, bevorzugt Chlor oder Fluor.

Ein $C_1$-$C_4$-Alkohol ist beispielsweise Methanol, Ethanol, Propanol, Isopropanol, Butanol oder Isobutanol, bevorzugt Methanol oder Ethanol, besonders bevorzugt Methanol.

Für das Reaktionsmedium werden 5 bis 100 Mol, bevorzugt 8 bis 80 Mol, besonders bevorzugt 10 bis 60 Mol, des Alkohols pro Mol der aromatischen Nitroverbindung eingesetzt,

Die Schwefelsäuremenge für das Reaktionsmedium beträgt 0,8 bis 2,5 Mol, bevorzugt 0,9 bis 1,5 Mol, besonders bevorzugt 1 bis 1,3 Mol, pro Mol der aromatischen Nitroverbindung.

Als erhöhte Temperatur gilt der Bereich von 50 bis 110°C, bevorzugt 60 bis 100°C, besonders bevorzugt 70 bis 90°C.

Es ist ein besonderes Kennzeichen des erfindungsgemäßen Verfahrens, daß es unter einem erhöhten Druck durchgeführt wird, Hierzu wird in einem Autoklaven, einem Druckkessel, einem Druckrohr oder einem ähnlichen druckfesten Reaktionsapparat, der dem Fachmann grundsätzlich bekannt ist, gearbeitet. Als erhöhter Druck sei ein solcher von mehr als 0,3 MPa (3 bar) bis zu 5 MPa (50 bar) genannt. An diesem erhöhten Druck ist der Wasserstoff-Partialdampfdruck mit mindestens 0,3 MPa (3 bar) beteiligt, kann aber auch den Gesamtdruck von bis zu 5 MPa (50 bar) ausmachen. Die Differenz zwischen 0,3 MPa (3 bar) Wasserstoff-Partialdampfdruck und dem Gesamtdruck von mehr als 0,3 MPa (3 bar) ist im allgemeinen der Eigendruck des Reaktionssystems, also im wesentlichen der Dampfdruck des Alkohols und (in geringerem Maße) der Nitroverbindung. Hinzu tritt beispielsweise bei Vorlage der aromatischen Nitroverbindung und des Reaktionsmediums in einem Autoklaven, Spülen desselben mit einem Inertgas, wie Stickstoff, Edelgase usw., Verschließen des Autoklaven und Erwärmen auf die gewünschte Reaktionstemperatur der sich erhöhende Druck im Gasraum des Autoklaven oberhalb der flüssigen Phase, bevor der zur Reduktion erforderliche Wasserstoff im erfindungsgemäßen (Partial-)Druck aufgedrückt wird. In bevorzugter Weise beträgt der Wasserstoff-Partialdruck mehr als 0,6 bis 10 MPa (6 bis 100 bar).

Als Katalysator für das erfindungsgemäße Verfahren kommen Edelmetalle der Platingruppe, insbesondere Platin selbst, in Frage. Das Platinmetall, bevorzugt Platin selbst, wird mit oder ohne Träger verwendet. Träger können beispielsweise Silikagel, Aluminiumoxid oder Kohle, bevorzugt Kohle, sein. Die Metallbelegung des Trägers beträgt 0,05-8 Gew.-%, bevorzugt 0,1-6 Gew.-%, besonders bevorzugt 0,25-5 Gew.-% des Gesamtkatalysators. Eine auf den Träger gebrachte Verbindung des Platinmetalls wird vor oder während der Reaktion zum Metall reduziert. Das Platinmetall wird ohne Desaktivator eingesetzt. Desgleichen werden keine Verbindungen eines Platinmetalls auf einen Träger gebracht, die ihrerseits Desaktivatoren darstellen. So werden insbesondere keine Schwefelverbindungen, wie Thiophen oder Dimethylsulfoxid, mitbenutzt.

Der Katalysator mit oder ohne Träger wird in einer solchen Menge eingesetzt, daß 0,001-0,3 Gew.-%, bevorzugt 0,005-0,1 Gew.-%, besonders bevorzugt 0,01-0,1 Gew.-% des Platinmetalls, bezogen auf die umzusetzende Nitroverbindung, vorliegen.

Das erfindungsgemäße Verfahren kann mit einem Nitrobenzol oder einem 1-Nitro-naphthalin durchgeführt werden, das gemäß Formel (I) substituiert sein kann. In bevorzugter Weise wird es mit einem gegebenenfalls substituierten Nitrobenzol durchgeführt.

In bevorzugter Weise werden dabei p-Methoxy- oder p-Ethoxy-aniline der Formel

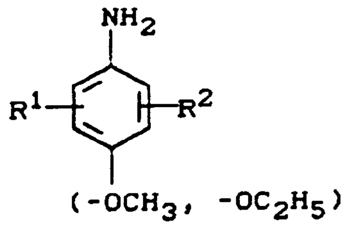

(III)

durch Umsetzung von Nitrobenzolen der Formel

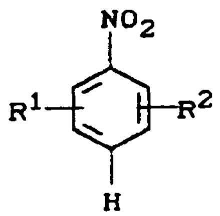

(IV)

wobei in beiden Formeln

$R^1$ und $R^2$ unabhängig voneinander Wasserstoff, $C_1$-$C_4$-Alkyl, $C_1$-$C_4$-Alkoxy oder Halogen bedeuten,

in Gegenwart von Methanol oder Ethanol unter den sonstigen oben angeführten Bedingungen erhalten.

In weiterhin bevorzugter Weise wird das erfindungsgemäße Verfahren mit einer aromatischen Nitroverbindung der Formel (I) durchgeführt, in der $R^2$ die Bedeutung Wasserstoff hat.

Ganz besonders wichtig ist die erfindungsgemäße Umsetzung von o- und m-Nitrotoluol bzw. Nitrobenzol.

Es hat sich weiterhin als vorteilhaft erwiesen, das gesamte Reaktionsgemisch nicht nur zur Steigerung der Reaktionsgeschwindigkeit, sondern auch zur weiteren Steigerung der Selektivität bezüglich des gewünschten p-Alkoxy-aminoaromaten während der erfindungsgemäßen Umsetzung in verstärktem Maße umzuwälzen. Dies kann durch eine erhöhte Rührgeschwindigkeit, durch eine erhöhte Hubfrequenz bei Hubautoklaven, durch eine erhöhte Schüttelgeschwindigkeit bei Schüttelautoklaven, durch verstärktes Umpumpen des unter erhöhtem Druck stehenden Wasserstoffs oder durch andere gleichwirkende Maßnahmen erzielt werden.

Zur Aufarbeitung wird vom Katalysator abfiltriert und in einer dem Fachmann bekannten Weise durch Destillation, Kristallisation, Extraktion oder Chromatographie aufgearbeitet.

Im erfindungsgemäßen Verfahren werden die in p-Stellung durch $C_1$-$C_4$-Alkoxy substituierten aromatischen Amine der Formel (I) in hohen Ausbeuten bei stark verkürzter Reaktionszeit erhalten. Der in nicht desaktivierter Form benutzte Katalysator kann in besonders einfacher Weise zurückgewonnen und wiederholt eingesetzt werden.

Beispiel 1

Ein Gemisch aus 45,7 g 3-Nitro-toluol (0,333 mol), 40 g $H_2SO_4$ (98 %), 577 g Methanol und 2 g Pt-Kohle-Katalysator (Gehalt 5 % Pt) wurde in einem Emailleautoklaven bei 80°C und einem $H_2$-Druck von 0,8 MPa (8 bar) hydriert. Die $H_2$-Aufnahme war nach 60 min. beendet. Das Reaktionsgemisch wurde durch Hochdruckflüssigkeitschromatographie analysiert und seine Zusammensetzung durch Vergleich mit Eichsubstanzen quantifiziert. Die Angaben finden sich in Tabelle 1.

Beispiel 2

Durchführung wie in Beispiel 1, jedoch bei 1,5 MPa (15 bar) $H_2$ (s. Tab. 1).

Beispiel 3

Durchführung wie in Beispiel 1, jedoch bei 2,5 MPa (25 bar) $H_2$ (s. Tab. 1).

Beispiel 4 (zum Vergleich)

Durchführung wie in Beispiel 1, jedoch bei 0,05 MPa (0,5 bar) $H_2$ (s. Tab. 1).

Beispiel 5

In einem 1,5 l-Emailleautoklaven wurden 450 g Methanol, 36,3 g (0,3 mol) Nitrobenzol, 35 g $H_2SO_4$ (98 %) und 0,2 g Pt/C-Katalysator (Gehalt 5 % Pt) vorgelegt. Unter starkem Rühren wurde auf 90°C erhitzt und 2 MPa (20 bar) $H_2$ aufgedrückt. Die Hydrierung war nach 35 min beendet. Nach Abfiltrieren des Katalysators wurde das Reaktionsgemisch wie in Beispiel 1 untersucht. Die Angaben finden sich in Tabelle 2.

Beispiel 6

Durchführung wie in Beispiel 5, jedoch bei 1 MPa (10 bar) $H_2$ (s. Tab. 2).

Beispiel 7 (zum Vergleich)

Durchführung wie im Beispiel 5, jedoch bei 0,13 MPa (1,3 bar) $H_2$ und 3 g des 5 %igen Pt/C-Katalysators (s. Tab. 2)

Beispiel 8

In einem 1 l Glasautoklaven wurden 350 g Methanol, 21,2 g (0,15 mol) 2-Fluornitrobenzol, 20 g Schwefelsäure (98 %) und 2 g Pt/C-Katalysator (Gehalt 1 % Pt) vorgelegt.

Unter starkem Rühren wurde auf 90°C erhitzt und 0,6 MPa (6 bar) Wasserstoff aufgedrückt.

Nach 1 Stunde wurde vom Katalystor abfiltriert und das Reaktionsgemisch am Rotationsverdampfer eingeengt. Der Rückstand wurde mit 50 ml $H_2O$ und 70 ml 10 %iger NaOH versetzt, 3 mal mit 100 ml Toluol extrahiert, die Toluolphase am Rotationsverdampfer eingeengt und das Produkt destilliert. Ausbeute: 9,5 g 2-Fluor-4-methoxy-anilin (45 % der theor. Ausbeute).

Tabelle 1 (Beispiele 1-4)

| Beispiel | $H_2$-Partialdruck [MPa/bar] | o-Kresidin [%] | m-Toluidin [%] | 2-Methyl-4-amino-phenol [%] | Reaktionszeit [min] | Edukt |
|---|---|---|---|---|---|---|
| 1 | 0,8/8,0 | 59,3 | 18,5 | 14,3 | 60 | 0 |
| 2 | 1,5/15,0 | 58,8 | 19,7 | 14,0 | 15-30 | 0 |
| 3 | 2,5/25,0 | 54,5 | 23,8 | 15,3 | 10 | 0 |
| 4 (Vgl.) | 0,05/0,5 | 56,3 | 6,5 | 13,6 | 510 | 8,5 |

Tabelle 2 (Beispiele 5-7)

| Beispiel | $H_2$-Partial-Druck [MPa/bar] | p-$NH_2$-anisol (%) | o-$NH_2$-anisol (%) | p-$NH_2$-phenol (%) | Anilin (%) | Reaktions-zeit (min) |
|---|---|---|---|---|---|---|
| 5 | 2/20 | 66,4 | 12,1 | 9,2 | 10,2 | 35 |
| 6 | 1/10 | 64,9 | 11,6 | 8,6 | 12,9 | 60 |
| 7 (Vgl.) | 0,13/1,3 | 56,4 | 10,2 | 8,0 | 23,5 | 230 |

## Patentansprüche

1. Verfahren zur Herstellung von in p-Stellung durch $C_1$-$C_4$-Alkoxy substituierten aromatischen Aminen der Formel

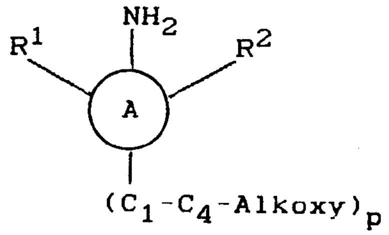

,

in der

| | |
|---|---|
| A | für den Benzol- oder den Naphthalinkern steht, |
| p | die p-Stellung zur Aminogruppe anzeigt und |
| $R^1$ und $R^2$ | unabhängig voneinander Wasserstoff, $C_1$-$C_4$-Alkyl, $C_1$-$C_4$-Alkoxy oder Halogen bedeuten, |

durch katalytische Hydrierung einer aromatischen Nitroverbindung in einem Reaktionsmedium aus Schwefelsäure und einem $C_1$-$C_4$-Alkohol bei erhöhter Temperatur, dadurch gekennzeichnet, daß als Nitroverbindung eine der Formel

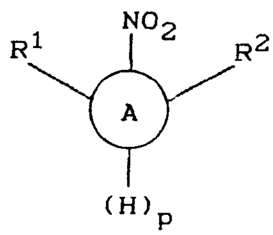

,

in der A, p, $R^1$ und $R^2$ die obige Bedeutung haben,
unter erhöhtem Druck, von dem der Wasserstoff-Partialdampfdruck 0,3 bis 10 MPa (3 bis 100 bar) beträgt, in Gegenwart nicht desaktivierter Katalysatoren aus der Platinmetall-Gruppe umgesetzt wird, wobei 5 bis 100 Mol des Alkohols und 0,8 bis 2,5 Mol Schwefelsäure, jeweils pro Mol der aromatischen Nitroverbindung, eingesetzt werden.

2. Verfahren nach Anspruch 1, dadurch gekennzeichnet, daß in p-Stellung durch $C_1$-$C_2$-Alkoxy, bevorzugt durch Methoxy, substituiert aromatische Amine durch Umsetzung in einem Reaktionsmedium, das einen $C_1$-$C_2$-Alkohol, bevorzugt Methanol, enthält, hergestellt werden.

3. Verfahren nach Anspruch 1, dadurch gekennzeichnet, daß die Alkoholmenge 5 bis 100 Mol, bevorzugt 8 bis 80 Mol, besonders bevorzugt 10 bis 60 Mol, pro Mol der aromatischen Nitroverbindung beträgt.

4. Verfahren nach Anspruch 1, dadurch gekennzeichnet, daß die Schwefelsäuremenge 0,8 bis 2,5 Mol, bevorzugt 0,9 bis 1,5 Mol, besonders bevorzugt 1 bis 1,3 Mol, pro Mol der aromatischen Nitroverbindung beträgt.

5. Verfahren nach Anspruch 1, dadurch gekennzeichnet, daß die erhöhte Temperatur 50 bis 110°C, bevorzugt 60 bis 100°C, besonders bevorzugt 70 bis 90°C, beträgt.

6. Verfahren nach Anspruch 1, dadurch gekennzeichnet, daß der Wasserstoff-Partialdruck mehr als 0,6 bis 10 MPa (6 bis 100 bar), beträgt.

7. Verfahren nach Anspruch 2, dadurch gekennzeichnet, daß p-Methoxy- oder p-Ethoxy-aniline der Formel

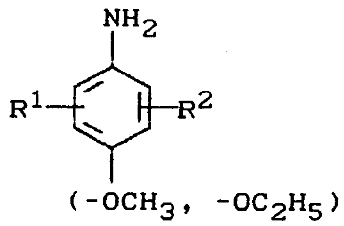

durch Umsetzung von Nitrobenzolen der Formel

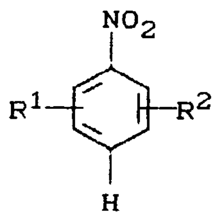
,

wobei in beiden Formeln

$R^1$ und $R^2$ unabhängig voneinander Wasserstoff, $C_1$-$C_4$-Alkyl, $C_1$-$C_4$-Alkoxy oder Halogen bedeuten,

in Gegenwart von Methanol oder Ethanol erhalten werden.

8. Verfahren nach Anspruch 1, dadurch gekennzeichnet, daß das Reaktionsgemisch während der Umsetzung durch eine erhöhte Rührgeschwindigkeit, durch eine erhöhte Hubfrequenz oder Schüttelgeschwindigkeit bei Hub- bzw. Schütelautoklaven, durch verstärktes Umpumpen des unter erhöhtem Druck stehenden Wasserstoffs oder durch andere gleichwirkende Maßnahmen verstärkt umgewälzt wird.

## Claims

1. Process for the preparation of aromatic amines substituted in the p position by $C_1$-$C_4$-alkoxy and having the formula

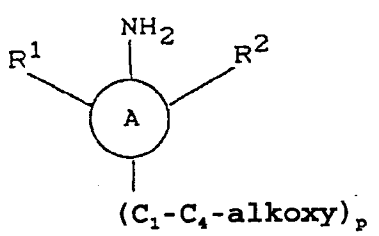
,

in which

A represents the benzene or naphthalene nucleus,

p indicates the p position to the amino group and

$R^1$ and $R^2$ independently of one another denote hydrogen, $C_1$-$C_4$-alkyl, $C_1$-$C_4$-alkoxy or halogen,

by catalytic hydrogenation of an aromatic nitro compound in a reaction medium comprising sulphuric acid and a $C_1$-$C_4$-alcohol at elevated temperature, characterized in that a nitro compound of the formula

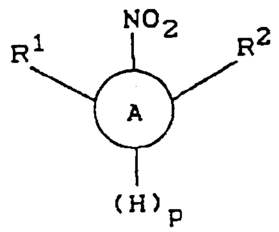

,

in which A, p, $R^1$ and $R^2$ have the above meaning,
is reacted at elevated pressure, of which the hydrogen partial pressure is from 0.3 to 10 MPa (from 3 to 100 bar), in the presence of non-deactivated catalysts from the platinum metal group, where from 5 to 100 mol of the alcohol and from 0.8 to 2.5 mol of sulphuric acid are used per mole of the aromatic nitro compound.

2. Process according to Claim 1, characterized in that aromatic amines substituted in the p position by $C_1$-$C_2$-alkoxy, preferably by methoxy, are prepared by reaction in a reaction medium containing a $C_1$-$C_2$-alcohol, preferably methanol.

3. Process according to Claim 1, characterized in that the amount of alcohol is from 5 to 100 mol, preferably from 8 to 80 mol, particularly preferably from 10 to 60 mol, per mole of the aromatic nitro compound.

4. Process according to Claim 1, characterized in that the amount of sulphuric acid is from 0.8 to 2.5 mol, preferably from 0.9 to 1.5 mol, particularly preferably from 1 to 1.3 mol, per mole of the aromatic nitro compound.

5. Process according to Claim 1, characterized in that the elevated temperature is from 50 to 110°C, preferably from 60 to 100°C, particularly preferably from 70 to 90°C.

6. Process according to Claim 1, characterized in that the hydrogen partial pressure is greater than from 0.6 to 10 MPa (from 6 to 100 bar).

7. Process according to Claim 2, characterized in that p-methoxy- or p-ethoxy-anilines of the formula

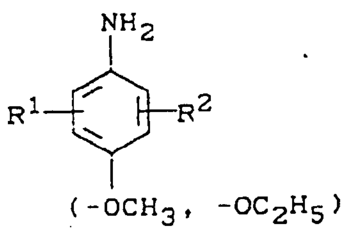

are obtained by reaction of nitrobenzenes of the formula

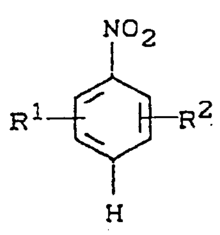

,

where in both formulae
$R^1$ and $R^2$ independently of one another denote hydrogen, $C_1$-$C_4$-alkyl, $C_1$-$C_4$-alkoxy or halogen, in the presence of methanol or ethanol.

8. Process according to Claim 1, characterized in that the reaction mixture is more strongly agitated during

the reaction by an increased stirring rate, by an increased lifting frequency or rocking rate for autoclaves with lifting mixers or rocking autoclaves respectively, by increased pump circulation of the hydrogen which is at elevated pressure or by other measures having a similar effect.

## Revendications

1. Procédé de préparation d'amines aromatiques substituées en position para par un groupe alcoxy en $C_1$-$C_4$ et répondant à la formule:

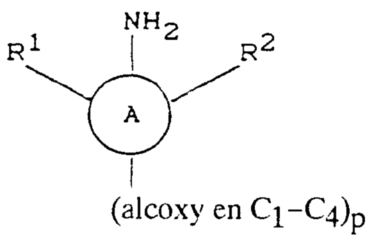

dans laquelle

| | |
|---|---|
| A | représente un noyau benzénique ou naphtalénique, |
| p | indique la position para par apport au groupe amino et |
| $R^1$ et $R^2$ | représentent chacun, indépendamment l'un de l'autre, l'hydrogène, un groupe alkyle en $C_1$-$C_4$, alcoxy en $C_1$-$C_4$ ou un halogène, |

par hydrogénation catalytique d'un dérivé aromatique nitré dans un milieu de réaction consistant en acide sulfurique et un alcool en $C_1$-$C_4$, à température élevée, caractérisé en ce que le dérivé nitré mis en oeuvre est un dérivé de formule:

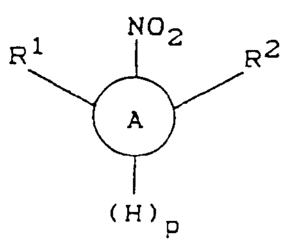 ,

dans laquelle
A, p, $R^1$ et $R^2$ ont les significations indiquées ci-dessus,
on opère sous forte pression, dans laquelle la pression partielle d'hydrogène représente de 0,3 à 10 MPa (3 à 100 bar), en présence de catalyseurs non désactivés du groupe des métaux du platine, et on met en oeuvre de 5 à 100 mol de l'alcool et de 0,8 à 2,5 mol d'acide sulfurique, dans les deux cas pour 1 mol du dérivé aromatique nitré.

2. Procédé selon la revendication 1, caractérisé en ce que l'on prépare des amines aromatiques substituées en position para par un groupe alcoxy en $C_1$-$C_2$, de préférence par un groupe méthoxy, par réaction dans un milieu contenant un alcool en $C_1$-$C_2$, de préférence le méthanol.

3. Procédé selon la revendication 1, caractérisé en ce que la quantité de l'alcool est de 5 à 100 mol, de préférence de 8 à 80 mol et dans les meilleures conditions de 10 à 60 mol par mole du dérivé aromatique nitré.

4. Procédé selon la revendication 1, caractérisé en ce que la quantité d'acide sulfurique est de 0,8 à 2,5 mol, de préférence de 0,9 à 1,5 mol, et dans les meilleures conditions de 1 à 1,3 mol par mole du dérivé aromatique nitré.

5. Procédé selon la revendication 1, caractérisé en ce que la température élevée en question est une température de 50 à 110°C, de préférence de 60 à 100°C et dans les meilleures conditions de 70 à 90°C.

6. Procédé selon la revendication 1, caractérisé en ce que la pression partielle d'hydrogène est supérieure à 0,6 MPa et peut aller jusqu'à 10 MPa (6 à 100 bar).

7. Procédé selon la revendication 2, caractérisé en ce que l'on obtient des p-méthoxy- ou p-éthoxy-anilines de formule :

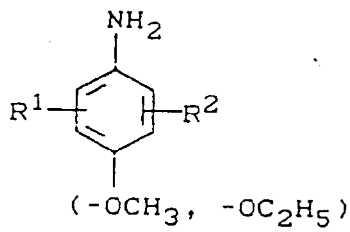

par conversion de nitrobenzènes de formule :

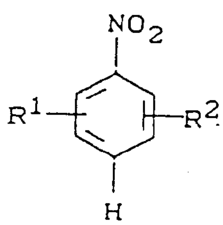

,

dans lesquelles
$R^1$ et $R^2$ représentent chacun, indépendamment l'un de l'autre, l'hydrogène, un groupe alkyle en $C_1$-$C_4$, alcoxy en $C_1$-$C_4$ ou un halogène,
en présence de méthanol ou d'éthanol.

8. Procédé selon la revendication 1, caractérisé en ce que, durant la réaction, le mélange de réacgion est soumis à circulation renforcée par augmentation de la vitesse d'agitation, de la fréquence de basculement ou de la vitesse des secousses dans les autoclaves à basculements ou à secousses, par circulation renforcée par pompe de l'hydrogène sous pression ou par d'autres moyens analogues.